# FASCICULE DE BREVET EUROPEEN

(11) **EP 3 999 004 B1**
(45) Date de publication et mention de la délivrance du brevet: **20.11.2024**
(21) Numéro de dépôt: 20742251.0
(22) Date de dépôt: 17.07.2020
(51) Int. Cl.: A61F 9/008, A61B 18/20, A61B 18/22

(54) **APPAREIL DE DECOUPE A COUPLEUR OPTIQUE INCLUANT UN CORRECTEUR DE POLARISATION**
SCHNEIDVORRICHTUNG MIT OPTISCHEM KOPPLER MIT POLARISATIONSKORREKTOR
CUTTING DEVICE WITH OPTICAL COUPLER INCLUDING A POLARISATION CORRECTOR

(30) Priorité: 19.07.2019 FR 1908208
(43) Date de publication de la demande: 25.05.2022
(73) Titulaire: Keranova, 42000 Saint Etienne (FR)
(72) Inventeur: BAUBEAU, Emmanuel, 42000 Saint Etienne (FR); NADOLNY, Sylvie, 42000 Saint Etienne (FR)
(74) Mandataire: Be IP
(86) Numéro de dépôt international: PCT/EP2020/070302
(87) Numéro de publication internationale: WO 2021/013734

(56) Documents cités:
- FR-A1- 3 026 940
- US-A1- 2017 340 483

## Description

### DOMAINE TECHNIQUE

La présente invention concerne le domaine technique du traitement de pathologies oculaires réalisé en utilisant un laser femtoseconde, et plus particulièrement celui de la chirurgie ophtalmologique pour notamment des applications de découpes de cornées, ou de cristallins.

L'invention concerne un dispositif de découpe d'un tissu humain ou animal, tel qu'une cornée, ou un cristallin, au moyen d'un laser femtoseconde.

Par laser femtoseconde, on entend une source lumineuse, apte à émettre un faisceau LASER sous forme d'impulsions ultra-courtes, dont la durée est comprise entre 1 femtoseconde et 100 picosecondes, de préférence comprise entre 1 et 1000 femtosecondes, notamment de l'ordre de la centaine de femtosecondes.

### ART ANTERIEUR

On a déjà proposé de réaliser des opérations chirurgicales de l'œil au moyen d'un laser femtoseconde, telles que des opérations de découpes de cornées ou de cristallins.

Le document FR 3 026 940 décrit un appareil de découpe incluant une source LASER et un système de mise en forme. Le document US 2017/340483 décrit un système pour la chirurgie oculaire comprenant : un système LASER, un microscope opératoire, une unité de commande, un bâti, un premier bras articulé sur lequel est fixée une tête du microscope opératoire, et un deuxième bras articulé sur lequel est fixée une tête applicatrice du système LASER, les deux têtes pouvant être reliées l'une à l'autre.

Les demandes internationales PCT/EP2019/051872 et PCT/EP2019/051876 en date du 25 janvier 2019 déposées au nom de la Demanderesse décrivent un appareil de découpe d'un tissu humain ou animal, tel qu'une cornée, ou un cristallin.

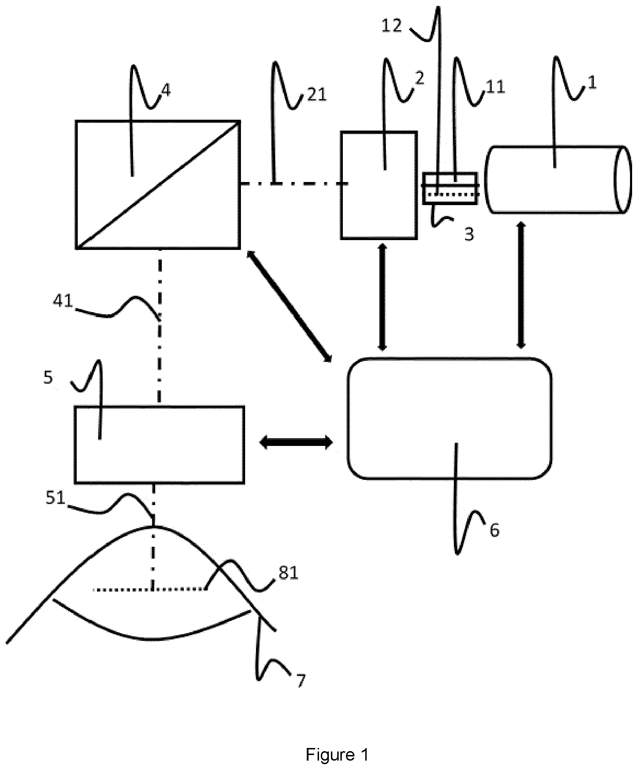

En référence à la figure 1, cet appareil comporte :
- une source LASER femtoseconde 1 pour émettre un faisceau LASER initial sous la forme d'impulsions,
- un système de mise en forme 2 - tel qu'un modulateur spatial de lumière (SLM) - positionné en aval de la source LASER 1, pour transformer le faisceau LASER initial en un unique faisceau LASER modulé en phase, le système de mise en forme étant apte à moduler la phase du front d'onde du faisceau LASER initial selon une consigne de modulation calculée pour répartir l'énergie du faisceau LASER en au moins deux points d'impact formant un motif dans un plan de focalisation 81,
- un coupleur optique 3 entre la source LASER femtoseconde 1 et le système de mise en forme 2, le coupleur optique 3 incluant une fibre optique à cristal photonique pour le filtrage du faisceau LASER 11 issu de la source LASER femtoseconde 1,
- un scanner optique de balayage 4, positionné en aval du système de mise en forme 2, pour déplacer le motif le long d'un chemin de déplacement prédéfini dans le plan de focalisation 81,
- un système optique de focalisation 5, positionné en aval du scanner optique de balayage 4, pour déplacer le plan de focalisation 81 du faisceau LASER modulé dans un plan de découpe du tissu 7 désiré,
- une unité de commande 6 permettant de piloter le système de mise en forme 2, le coupleur optique 3, le scanner optique de balayage 4 et le système optique de focalisation 5.

Avantageusement, ce dispositif de découpe peut être intégré dans un bras articulé. Plus précisément, le système de mise en forme 2, le scanner optique de balayage 4 et le système optique de focalisation 5 peuvent être montés dans un compartiment (dénommé dans la suite « *tête de travail »*) fixé à une extrémité libre du bras articulé, tandis que la source LASER 1 et l'unité de commande 6 peuvent être intégrées dans un caisson du bras articulé, le coupleur optique 3 s'étendant entre le caisson et la tête de travail pour propager le faisceau LASER 11 entre la source LASER 1 et le système de mise en forme 2.

Le coupleur optique 3 permet de simplifier le dispositif de découpe en facilitant la transmission du faisceau LASER 11 entre la source LASER 1 et le système de mise en forme 2 qui sont distants. En effet, à cause de son encombrement important, la source LASER 1 ne peut être positionnée dans la tête de travail du bras articulé.

Toutefois, lors de traversée de la fibre à cristal photonique, le faisceau LASER 11 issu de la source LASER 1 subit une variation de sa polarisation. Cette variation de polarisation dépend de la position et de l'orientation de la fibre à cristal photonique (qui dépend de la position et de l'orientation de la tête de travail relativement au caisson du bras articulé).

Or le système de mise en forme 2 étant sensible à la polarisation du faisceau LASER, cette variation de polarisation induit une diminution de la puissance du faisceau LASER modulé en sortie du système de mise en forme 2.

Un but de la présente invention est de proposer une solution technique permettant de maintenir toute la puissance du faisceau LASER modulé quelles que soient la position et l'orientation du système de mise en forme 2 relativement à la source LASER 1.

### EXPOSE DE L'INVENTION

A cet effet, l'invention propose un appareil de découpe d'un tissu humain ou animal, tel qu'une cornée, ou un cristallin, ledit appareil incluant :
- une source LASER pour émettre un faisceau LASER de traitement sous la forme d'impulsions,
- un système de mise en forme - tel qu'un modulateur spatial de lumière (SLM) - positionné en aval de la source LASER, pour transformer le faisceau LASER de traitement en un unique faisceau LASER de traitement modulé en phase, le système de mise en forme étant apte à moduler la phase du front d'onde du faisceau LASER de traitement selon une consigne de modulation calculée pour répartir l'énergie du faisceau LASER de traitement en au moins deux points d'impact formant un motif dans un plan de focalisation,
- un coupleur optique entre la source LASER et le système de mise en forme, le coupleur optique incluant une fibre optique à cristal photonique,
***remarquable en ce que*** l'appareil de découpe comprend en outre un correcteur de polarisation pour modifier la polarisation du faisceau LASER de traitement en amont du système de mise en forme, de sorte que la polarisation du faisceau LASER de traitement en entrée du système de mise en forme corresponde à une polarisation de référence souhaitée, le correcteur de polarisation étant monté en amont du système de mise en forme. Avantageusement, le correcteur de polarisation peut comprendre :
- des moyens pour mesurer une variation de polarisation entre une extrémité d'entrée du coupleur optique et une extrémité de sortie du coupleur optique, et
- des moyens pour modifier la polarisation du faisceau LASER de traitement en amont du coupleur optique de sorte à compenser la variation de la polarisation mesurée ;

La présence d'un correcteur de polarisation permet de modifier la polarisation du faisceau LASER initial en entrée du coupleur optique de sorte que la polarisation du faisceau LASER en sortie du coupleur optique soit sensiblement égale à une polarisation de référence souhaitée pour le faisceau LASER avant son introduction dans le système de mise en forme. Cette polarisation de référence souhaitée correspond à la polarisation du faisceau LASER permettant d'obtenir une puissance maximale pour le faisceau LASER modulé en sortie du système de mise en forme.

On entend, dans le cadre de la présente invention, par *« point d'impact »* une zone du faisceau LASER comprise dans son plan focal dans laquelle l'intensité dudit faisceau LASER est suffisante pour générer une bulle de gaz dans un tissu.

On entend, dans le cadre de la présente invention, par *« points d'impact adjacents »,* deux points d'impact disposés en regard l'un de l'autre et non séparés par un autre point d'impact. On entend par *« points d'impact voisins »* deux points d'un groupe de points adjacents entre lesquels la distance est minimale.

On entend, dans le cadre de la présente invention, par *« motif »* une pluralité de points d'impact LASER générés simultanément dans un plan de focalisation d'un faisceau LASER mis en forme - c'est-à-dire modulé en phase pour répartir son énergie en plusieurs spots distincts dans le plan de focalisation correspondant au plan de découpe du dispositif. Ainsi, le système de mise en forme permet de modifier le profil d'intensité du faisceau LASER dans le plan de la découpe, d'une manière à pouvoir améliorer la qualité ou bien la vitesse de la découpe en fonction du profil choisi. Cette modification de profil d'intensité est obtenue par modulation de la phase du faisceau LASER.

La modulation optique de phase peut être réalisée au moyen d'un masque de phase. L'énergie du faisceau LASER incident est conservée après modulation, et la mise en forme du faisceau est réalisée en agissant sur son front d'onde. La phase d'une onde électromagnétique représente la situation instantanée de l'amplitude d'une onde électromagnétique. La phase dépend aussi bien du temps que de l'espace. Dans le cas de la mise en forme spatiale d'un faisceau LASER, seules les variations dans l'espace de la phase sont considérées.

Le front d'onde est défini comme la surface des points d'un faisceau possédant une phase équivalente (i.e. la surface constituée des points dont les temps de parcours depuis la source ayant émis le faisceau sont égaux). La modification de la phase spatiale d'un faisceau passe donc par la modification de son front d'onde.

Cette technique permet de réaliser l'opération de découpe d'une manière plus rapide et plus efficace car elle met en oeuvre plusieurs spots LASER réalisant chacun une découpe et selon un profil contrôlé.

Le fait de positionner le coupleur optique incluant la fibre optique à cristal photonique entre le laser femtoseconde et le système de mise en forme permet d'éviter toute perturbation dans la mise en forme du faisceau laser réalisée par le système de mise en forme.

Des aspects préférés mais non limitatifs de l'appareil de découpe sont les suivants :
- le correcteur de polarisation peut être monté entre la source LASER et le système de mise en forme, ledit correcteur de polarisation étant apte à modifier la polarisation du faisceau LASER de traitement en amont du coupleur optique de sorte que la polarisation du faisceau LASER de traitement en aval du coupleur optique corresponde à la polarisation de référence souhaitée ;
- les moyens pour mesurer peuvent être optiquement connectés à l'extrémité de sortie du coupleur optique, lesdits moyens pour mesurer étant aptes à mesurer une variation de polarisation du faisceau LASER de traitement à partir d'un faisceau LASER de mesure généré par la source LASER, l'intensité du faisceau LASER de mesure étant inférieure à l'intensité du faisceau LASER de traitement ;
- les moyens pour mesurer peuvent comprendre :
   - un polariseur pour filtrer sélectivement un plan de polarisation du faisceau LASER de mesure, et
   - un analyseur de polarisation monté en aval du polariseur pour mesurer une information représentative de la polarisation du faisceau LASER de mesure en sortie du coupleur optique ;
- les moyens pour mesurer peuvent comprendre en outre un calculateur pour calculer, à partir de l'information mesurée par l'analyseur de polarisation, une variation de polarisation Δₚₒₗₐᵣᵢₛₐₜᵢₒₙ entre :
   - la polarisation du faisceau LASER de mesure émis par la source LASER, et
   - la polarisation du faisceau LASER de mesure reçu par l'analyseur de polarisation ;
- les moyens pour modifier la polarisation du faisceau LASER de traitement peuvent être disposés entre la source LASER et le coupleur optique, lesdits moyens pour modifier la polarisation étant aptes à pivoter le plan de polarisation du faisceau LASER de traitement en amont du coupleur optique d'un angle opposé à la variation de polarisation mesurée ;
- la polarisation de référence souhaitée peut être égale à la polarisation du faisceau LASER de traitement en sortie de la source LASER (i.e. préalablement à la traversée du correcteur de polarisation et du coupleur optique) ;
- l'appareil de découpe peut comprendre en outre :
   - un scanner optique de balayage, positionné en aval du système de mise en forme, pour déplacer le motif le long d'un chemin de déplacement prédéfini dans le plan de focalisation,
   - un système optique de focalisation, positionné en aval du scanner optique de balayage, pour déplacer le plan de focalisation du faisceau LASER modulé dans un plan de découpe du tissu désiré,
   - une unité de commande pour piloter la source LASER, le système de mise en forme, le coupleur optique, le scanner optique de balayage et le système optique de focalisation.

L'invention concerne également un dispositif de thérapie comportant un caisson et un bras articulé monté sur le caisson, le bras incluant plusieurs segments de bras reliés par des articulations, ***remarquable en ce que*** le dispositif de thérapie comprend en outre un appareil de découpe tel que décrit ci-dessus, le système de mise en forme, le scanner optique de balayage et le système optique de focalisation étant montés dans un segment d'extrémité du bras articulé, la source LASER et l'unité de commande étant intégrées au caisson.

Avantageusement, les moyens pour modifier la polarisation du faisceau LASER de traitement peuvent être intégrés au caisson.

### BREVE DESCRIPTION DES DESSINS

D'autres caractéristiques et avantages de l'invention ressortiront clairement de la description qui en est réalisée ci-après, à titre indicatif et nullement limitatif, en référence aux figures annexées, dans lesquelles :
- la figure 1 est une représentation schématique d'un montage incluant un appareil de découpe ;
- la figure 2 est une représentation schématique d'un dispositif de thérapie à bras robotisé articulé intégrant l'appareil de découpe illustré à la figure 1 ;
- la figure 3 est une représentation schématique d'un coupleur optique, d'un correcteur de polarisation et d'une unité de commande de l'appareil de découpe ;
- la figure 4 est une représentation schématique détaillée du coupleur optique et du correcteur de polarisation.

### EXPOSE DETAILLE DE L'INVENTION

L'invention concerne un appareil de découpe d'un tissu humain ou animal au moyen d'une source LASER femtoseconde. Dans la suite de la description, l'invention sera décrite, à titre d'exemple, pour la découpe d'une cornée d'un oeil humain ou animal, étant bien entendu que la présente invention peut être utilisée pour tout autre type de traitement d'un tissu oculaire.

### 1. Généralités

### 1.1. Appareil de découpe

Comme indiqué précédemment, l'appareil de découpe comprend :
- une source LASER femtoseconde 1 apte à émettre un faisceau LASER de traitement 11 sous la forme d'impulsions de haute intensité,
- un système de mise en forme 2 positionné en aval de la source LASER 1 pour moduler la phase du faisceau LASER de traitement 11 et obtenir un faisceau LASER unique modulé 21 dans lequel l'énergie du faisceau LASER de traitement 11 est répartie en une pluralité de points d'impact dans son plan focal, cette pluralité de points d'impact définissant un motif,
- un coupleur optique 3 entre la source LASER 1 et le système de mise en forme 2, le coupleur permettant la propagation du faisceau LASER de traitement 11 issu de la source LASER 1 vers le système de mise en forme 2,
- un scanner optique de balayage 4 en aval du système de mise en forme 2, le scanner optique de balayage 4 permettant d'orienter le faisceau LASER modulé 21 pour déplacer le motif le long d'un chemin de déplacement prédéfini par l'utilisateur dans un plan de focalisation 81,
- un système optique de focalisation 5 en aval du scanner optique de balayage 4, le système optique de focalisation permettant de déplacer le plan de focalisation 81 - correspondant au plan de découpe - du faisceau LASER dévié 41 issu du scanner optique de balayage 4,
- une unité de commande 6 permettant de piloter la source LASER 1, le système de mise en forme 2, le coupleur optique 3, le scanner optique de balayage 4 et le système optique de focalisation 5.

Le système de mise en forme 2 permet de moduler la phase du faisceau LASER de traitement 11 issu de la source LASER 1 pour former des pics d'intensité dans le plan focalisation 81, chaque pic d'intensité produisant un point d'impact respectif dans le plan focal correspondant au plan de découpe. Le système de mise en forme 2 est, selon le mode de réalisation illustré, un modulateur spatial de lumière à cristaux liquides, connu sous le sigle SLM, de l'acronyme anglais *« Spatial Light Modulator ».* D'une manière connue, le SLM met en oeuvre un masque de phase, c'est-à-dire une carte déterminant comment la phase du faisceau LASER de traitement doit être modifiée pour obtenir une répartition d'amplitude donnée dans son plan de focalisation 81. L'utilisation d'un tel système de mise en forme permet d'une part une diminution du temps de découpe du tissu biologique (en générant plusieurs points d'impacts simultanément), et d'autre part l'obtention de points d'impact sensiblement égaux (la forme, la position et le diamètre de chaque point étant contrôlés dynamiquement par un masque de phase calculé et affiché sur le système de mise en forme).

Le coupleur optique 3 permet la transmission du faisceau LASER de traitement 11 entre la source LASER 1 et le système de mise en forme 2. Le coupleur optique 3 comprend avantageusement une fibre optique. Cette fibre optique peut être une fibre à cristal photonique ou *« PCF »* (sigle de l'expression anglo-saxonne « *Photonic-CrystalFiber »*)*,* et notamment une fibre à cristal photonique à coeur creux. Une fibre à cristal photonique à coeur creux est particulièrement adaptée à la propagation d'impulsions LASER courtes de haute énergie. L'utilisation d'une telle fibre est donc avantageuse pour véhiculer de manière optimale le faisceau LASER de traitement 11 issu de la source LASER 1.

### 1.2. Dispositif de thérapie

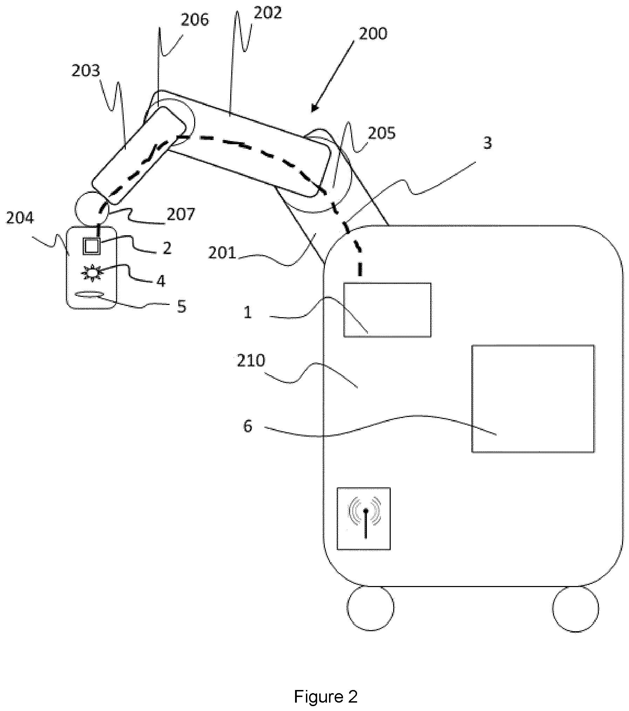

Grâce à l'utilisation d'un coupleur optique 3, l'appareil de découpe peut être monté dans un dispositif de thérapie incluant un bras articulé 200 et un caisson fixe 210 sur lequel est monté le bras articulé 200, tel qu'illustré à la figure 2. Toutefois, le lecteur appréciera que l'appareil de découpe n'est pas nécessairement monté dans un dispositif de thérapie incluant un bras et un caisson fixe. En particulier, le dispositif de thérapie peut être utilisé seul, c'est-à-dire sans être monté dans un bras articulé et un caisson.

Le bras 200 comprend plusieurs segments de bras 201-204 reliés par des articulations 205-207 (liaisons pivot ou rotule) motorisées pour permettre le déplacement automatique en rotation des différents segments 201-204 les uns par rapport aux autres.

Le bras 200 est apte à se déplacer entre :
- une position de repos (non représentée) facilitant son transport d'une salle d'intervention à une autre et/ou à l'intérieur d'une salle d'intervention, et
- une position de travail dans laquelle l'extrémité du bras 200 s'étend au droit du tissu oculaire à traiter.

Le système de mise en forme 2, le scanner optique de balayage 4 et le système optique de focalisation 5 peuvent être montés dans le segment d'extrémité 204 (i.e. *« tête de travail »*) du bras 200.

La source LASER 1 et l'unité de commande 6 peuvent être intégrées au caisson mobile 210 du dispositif de thérapie, le coupleur optique 3 s'étendant entre le caisson 210 et le segment d'extrémité 204 pour propager le faisceau LASER de traitement 11 issu de la source LASER 1 vers le système de mise en forme 2.

### 2. Correcteur de polarisation

### 2.1. Généralités

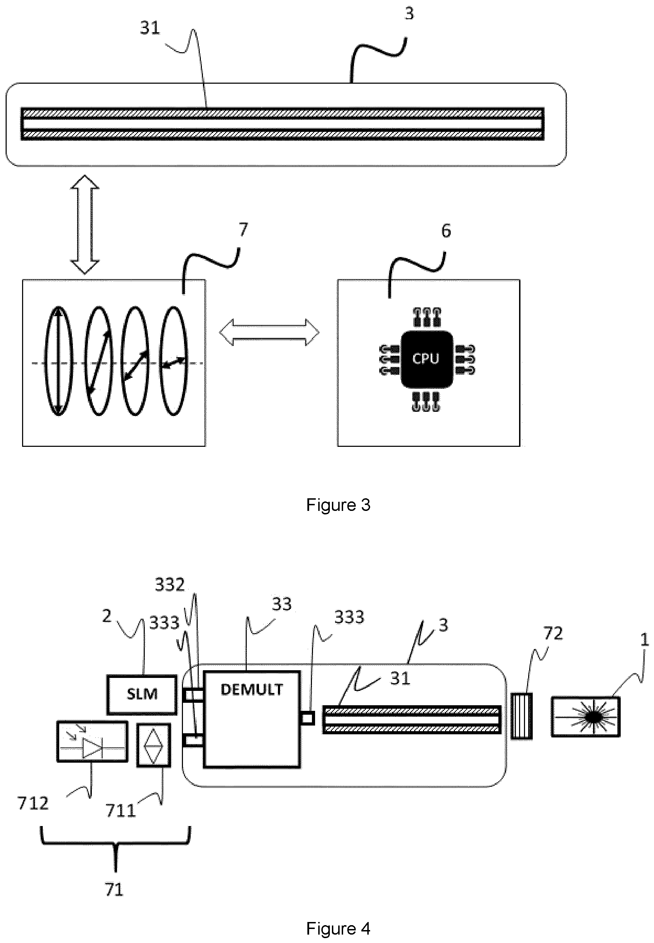

En référence à la figure 3, l'appareil de découpe comprend également un correcteur de polarisation 7.

Ce correcteur de polarisation 7 permet de modifier la polarisation du faisceau LASER de traitement 11 en entrée du coupleur optique 3 de sorte que la polarisation du faisceau LASER de traitement en sortie du coupleur optique 3 soit sensiblement égale à une polarisation de référence souhaitée pour le faisceau LASER de traitement avant son introduction dans le système de mise en forme 2.

En effet, comme indiqué précédemment, le système de mise en forme 2 permet de moduler la phase du faisceau LASER de traitement 11 issu de la source LASER 1 pour former des pics d'intensité dans le plan focalisation 81, chaque pic d'intensité produisant un point d'impact respectif dans le plan focal correspondant au plan de découpe.

La modulation en phase du front d'onde peut être vue comme un phénomène d'interférences en deux dimensions. Chaque portion du faisceau LASER de traitement 11 issu de la source 1 est retardée ou avancée par rapport au front d'onde initial afin que chacune de ces portions soit redirigée de façon à réaliser une interférence constructive en N points distincts dans le plan focal d'une lentille. Cette redistribution d'énergie en une pluralité de points d'impact n'a lieu que dans un seul plan (i.e. le plan de focalisation 81) et pas tout au long du chemin de propagation du faisceau LASER de traitement modulé.

Cependant, les systèmes de mise en forme 2 sont sensibles à la polarisation du faisceau LASER de traitement entrant. Par exemple, dans le cas d'un modulateur spatial de lumière à cristaux liquides, une modulation de phase « *pure »* est possible si la polarisation du front d'onde du faisceau LASER de traitement entrant dans le système de mise en forme 2 est alignée avec l'axe extraordinaire des cristaux liquides du modulateur spatial de lumière.

Ainsi, pour effectuer une modulation de phase *« pure »* du faisceau LASER de traitement 11 issu de la source LASER 1, il est préférable que la polarisation du faisceau LASER de traitement entrant dans le système de mise en forme 2 corresponde à une polarisation de référence souhaitée (par exemple dans le cas d'un modulateur spatial de lumière à cristaux liquides, il est préférable que la polarisation du faisceau LASER de traitement entrant dans le SLM soit alignée avec l'axe optique du cristal liquide).

Or, le déplacement de la tête de travail 204 pour atteindre la position de travail induit des changements de position et d'orientation du coupleur optique (et en particulier de la fibre optique du coupleur qui peut subir des torsions, etc.) qui peuvent modifier la polarisation du faisceau LASER de traitement entrant dans le système de mise en forme 2.

Cette modification de la polarisation du faisceau LASER de traitement entrant dans le système de mise en forme 2 peut dégrader la qualité du signal modulé en sortie du système de mise en forme 2.

Le correcteur de polarisation 7 permet de corriger la polarisation du faisceau LASER de traitement entrant dans le système de mise en forme 2 de sorte que la qualité du faisceau LASER de traitement modulé 21 sortant du système de mise en forme 2 soit optimale.

### 2.2. Eléments constituant le correcteur de polarisation

Le correcteur de polarisation 7 est connecté à l'unité de commande 6 pour permettre la transmission de données mesurées par le correcteur de polarisation 7 vers l'unité de commande 6, et pour permettre la transmission de signaux de contrôle émis par l'unité de commande 6 vers le correcteur de polarisation 7.

En référence à la figure 4, le correcteur de polarisation 7 comprend :
- des moyens 71 pour mesurer la variation de polarisation du faisceau LASER de traitement 11 entre l'entrée du coupleur optique 3 et la sortie du coupleur optique 3, et
- des moyens 72 pour modifier la polarisation du faisceau LASER de traitement 11 entrant dans le coupleur optique 3 de sorte à compenser cette variation mesurée de la polarisation du faisceau LASER entre l'entrée et la sortie du coupleur optique.

### 2.2.1. Moyens de mesure d'une variation de polarisation

Une fois le bras articulé 200 en position de travail, les moyens 71 de mesure permettent d'estimer les modifications de polarisation subies par le faisceau LASER de traitement 11 lors de son passage au travers du coupleur optique 3.

Pour mesurer les modifications de polarisation subies par le faisceau LASER de traitement 11, l'unité de commande 6 est programmée pour piloter la source LASER 1 de sorte qu'un faisceau LASER de mesure 12 soit généré préalablement à l'émission du faisceau LASER de traitement 11, l'intensité du faisceau LASER de mesure 12 étant très inférieure (notamment entre 10 et 1000 fois inférieur) à l'intensité du faisceau LASER de traitement 11.

Ainsi, pour mesurer les modifications de polarisation subies par le faisceau LASER de traitement 11, l'utilisation d'un faisceau LASER de mesure 12 (de faible intensité) issu de la source LASER 1 est privilégiée par rapport à l'utilisation d'un faisceau lumineux polarisé issu d'une source de lumière polarisée secondaire de faible intensité, telle qu'une diode électroluminescente associée à un polariseur linéaire.

Les inventeurs ont en effet découvert que des faisceaux lumineux de polarisation identiques issus de deux sources lumineuses différentes ne subissaient pas les mêmes modifications de polarisation lors de la traversée du coupleur optique 3.

Dans un mode de réalisation, les moyens 71 de mesure comprennent un polariseur linéaire 711 - tel qu'un polariseur basé sur un traitement diélectrique, ou un polariseur basé sur des matériaux biréfringents - et un analyseur de polarisation 712 - tel qu'une photodiode ou un phototransistor - en aval du polariseur linéaire 711.

Le polariseur linéaire 711 permet de filtrer sélectivement la lumière polarisée issue du coupleur optique 3. L'analyseur de polarisation 712 permet de mesurer une information représentative de la polarisation du faisceau LASER de mesure 12 issu du coupleur optique 3.

Les moyens 71 de mesure sont reliés optiquement à la sortie du coupleur optique 3 par l'intermédiaire d'un démultiplexeur 33 optique, qui peut être intégré au coupleur optique 3 ou au polariseur linéaire 711.

Plus précisément, le coupleur optique 3 comprend un démultiplexeur 33 optique monté à l'extrémité de la fibre à cristal photonique à coeur creux 31 la plus proche du système de mise en forme 2.

Le démultiplexeur 33 optique comporte :
- un canal d'entrée 333 optiquement connecté à la fibre à cristal photonique,
- un premier canal de sortie 331 optiquement connecté au système de mise en forme 2 pour permettre la transmission du faisceau LASER de traitement 11 issu de la source LASER 1 vers le système de mise en forme 2,
- un deuxième canal de sortie 332 optiquement connecté aux moyens 71 de mesure pour permettre la transmission du faisceau LASER de mesure 12 vers les moyens 71 de mesure.

Le démultiplexeur 33 optique permet de sélectionner le canal de sortie 331, 332 sur lequel le faisceau traversant le canal d'entrée 333 (i.e. faisceau LASER de mesure ou de traitement) est transmis afin de diriger ce faisceau soit vers le système de mise en forme 2, soit les moyens 71 de mesure.

Le principe de fonctionnement des moyens 71 pour mesurer la variation de polarisation du faisceau LASER entre l'entrée du coupleur optique 3 et la sortie du coupleur optique 3 est le suivant. On suppose ici que le bras articulé 200 est en position de travail, c'est-à-dire que l'extrémité 204 du bras 200 (incluant le système de mise en forme 2, le scanner optique de balayage 4 et le système optique de focalisation 5) s'étend au droit du tissu oculaire à traiter.

Préalablement à l'émission par la source LASER 1 du faisceau LASER de traitement 11 pour le traitement du tissu, la source LASER 1 est activée par l'unité de commande 6 pour émettre un faisceau LASER de mesure 12 de faible intensité. Le faisceau LASER de mesure 12 pénètre dans le coupleur optique 3 et traverse la fibre à cristal photonique 31.

Lors de la traversée de la fibre optique à cristal photonique 31, la polarisation du faisceau LASER de mesure 12 subit des variations (par exemple rotation du plan de polarisation du fait de torsions de la fibre optique à cristal photonique 31).

Le faisceau LASER de mesure 12 traverse ensuite le canal d'entrée 333 du démultiplexeur 33 et est basculé vers le deuxième canal de sortie 332 du démultiplexeur 33. Les moyens 71 de mesure captent le faisceau LASER de mesure 12. L'analyseur de polarisation 712 mesure une information représentative de la polarisation du faisceau LASER de mesure 12 reçu.

L'information mesurée par l'analyseur de polarisation 712 est transmise à un calculateur des moyens 71 de mesure (qui peut être intégré ou non à l'unité de commande 6). A partir de cette information mesurée, et connaissant la polarisation du faisceau LASER de mesure 12 émis par la source LASER 1, le calculateur est apte à calculer une variation de polarisation Δₚₒₗₐᵣᵢₛₐₜᵢₒₙ entre :
- la polarisation du faisceau LASER de mesure 12 émis par la source LASER 1, et
- la polarisation du faisceau LASER de mesure 12 reçu par le l'analyseur de polarisation712.

Cette variation de polarisation calculée est utilisée par le calculateur pour générer un signal de compensation permettant de corriger la polarisation du faisceau LASER de traitement 11 émis par la source LASER 1 afin que la polarisation du faisceau LASER de traitement 11 en sortie du coupleur optique 3 corresponde à une polarisation de référence souhaitée pour le faisceau LASER de traitement avant son introduction dans le système de mise en forme 2.

Ce signal de compensation est transmis aux moyens 72 pour modifier la polarisation du faisceau LASER.

### 2.2.2. Moyens de modification de la polarisation

Les moyens 72 pour modifier la polarisation du faisceau LASER de traitement permettent de corriger la variation de polarisation Δₚₒₗₐᵣᵢₛₐₜᵢₒₙ mesurée par les moyens 71 de mesure.

Les moyens 72 pour modifier la polarisation peuvent être montés en aval du coupleur optique 3. Dans ce cas, les moyens 72 pour modifier la polarisation sont intégrés dans le segment d'extrémité 204 (i.e. « *tête de travail »*) du bras 200. Ceci peut induire des problèmes d'encombrement.

En variante, les moyens 72 pour modifier la polarisation peuvent être montés entre la source LASER 1 et le coupleur optique 3. Ceci permet d'intégrer les moyens 72 pour modifier la polarisation dans le caisson 210 du dispositif de thérapie (ce qui limite les problèmes d'encombrement). Dans ce cas, les moyens 72 pour modifier la polarisation permettent de générer un faisceau LASER de polarisation corrigée en entrée du coupleur optique 3, de sorte que la polarisation du faisceau LASER en sortie du coupleur optique 3 corresponde à une polarisation de référence souhaitée

Plus précisément, la polarisation du faisceau LASER de traitement 11 en sortie de la source LASER 1 correspond à une polarisation optimale, c'est-à-dire à la polarisation de référence souhaitée pour le faisceau LASER de traitement avant son introduction dans le système de mise en forme 2.

La traversée de la fibre optique à cristal photonique 31 modifie cette polarisation.

Les moyens 72 pour modifier la polarisation permettent de compenser les modifications de polarisation subies par le faisceau LASER de traitement 11 lors de la traversée de la fibre optique à cristal photonique 31.

Ainsi, la polarisation du faisceau LASER de traitement 11 en sortie du coupleur optique 3 correspond à la polarisation optimale du faisceau LASER 11 de traitement en sortie de la source LASER 1.

Les moyens 72 pour modifier la polarisation peuvent comprendre une cellule électro-optique dont le rôle est de faire tourner le plan de polarisation du faisceau LASER de traitement 11 issu de la source LASER 1 sous l'action d'une tension électrique de commande. Cette cellule peut être de l'un des types connus de l'homme du métier pour agir sur la lumière. Par exemple cette cellule peut être une cellule à cristal liquide (un tel cristal est particulièrement facile à commander avec une dépense d'énergie faible) ou de préférence une lame à retard tournante - en particulier une lame demi-onde tournante - qui présente l'avantage d'être beaucoup moins onéreuse qu'une cellule à cristal liquide.

Le principe de fonctionnement des moyens 72 pour modifier la polarisation est le suivant. On suppose ici que les moyens 72 pour modifier la polarisation sont positionnés entre la source LASER 1 et le coupleur optique 3.

Une fois le signal de compensation estimé par le calculateur, ce signal de compensation est envoyé aux moyens 72 pour modifier la polarisation afin de les configurer.

Lors de la traversée des moyens 72 pour modifier la polarisation, le plan de polarisation du faisceau LASER de traitement 11 issu de la source LASER 1 subit une rotation dans le sens opposé à la variation de polarisation Δₚₒₗₐᵣᵢₛₐₜᵢₒₙ mesurée. On obtient un faisceau LASER de polarisation corrigée.

Par exemple, si la variation de polarisation Δₚₒₗₐᵣᵢₛₐₜᵢₒₙ mesurée correspond à une rotation du faisceau lumineux polarisé d'un angle de 45° dans le sens trigonométrique, le plan de polarisation du faisceau LASER de traitement 11 issu de la source LASER 1 est pivoté d'un angle de 45° dans le sens rétrograde (i.e. angle de -45° dans le sens trigonométrique).

Ainsi, en sortie du coupleur optique 3, la polarisation du faisceau LASER de traitement 11 correspond à la polarisation du faisceau LASER de traitement 11 en sortie de la source LASER 1.

Le correcteur de polarisation 7 permet de modifier la polarisation du faisceau LASER de traitement 11 avant son introduction dans le système de mise en forme 2. Cette solution permet de maintenir toute la puissance du faisceau LASER modulé 21 en sortie du système de mise en forme 2 quelle que soit la position et l'orientation du segment d'extrémité 204 (i.e. « *tête de travail »*) du bras articulé 200.

### 3. Conclusions

L'invention permet de disposer d'un outil de découpe efficace et précis. La modulation reconfigurable du front d'onde du faisceau LASER permet de générer de multiples points d'impact simultanés ayant chacun une taille et une position contrôlées dans le plan de focalisation 81. Ces différents points d'impact forment un motif dans le plan focal 71 du faisceau LASER modulé.

L'utilisation d'un coupleur optique 3 incluant une fibre à cristal photonique 31 à coeur creux permet de réduire la distance entre les différents points d'impacts formant le motif. En effet, en limitant le phénomène d'étalement du spectre lumineux, le coupleur optique 3 incluant une fibre à cristal photonique 31 à coeur creux permet de rendre le faisceau LASER modulé en phase plus propre.

La présence du correcteur de polarisation 7 permet de compenser les variations de polarisation subies par le faisceau LASER de traitement 11 lors de la traversée du coupleur optique 3 de sorte que la polarisation du faisceau LASER de traitement 11 en entrée du système de mise en forme 2 corresponde à la polarisation souhaitée en entrée de dispositif de mise en forme.

L'invention est définie dans les revendications suivantes.

## Revendications

1. Appareil de découpe d'un tissu humain ou animal, tel qu'une cornée, ou un cristallin, ledit appareil incluant :
- une source LASER (1) pour émettre un faisceau LASER de traitement (11) sous la forme d'impulsions,
- un système de mise en forme (2) - tel qu'un modulateur spatial de lumière (SLM) - positionné en aval de la source LASER (1), pour transformer le faisceau LASER de traitement (11) en un unique faisceau LASER de traitement modulé en phase, le système de mise en forme (2) étant apte à moduler la phase du front d'onde du faisceau LASER de traitement (11) selon une consigne de modulation calculée pour répartir l'énergie de l'unique faisceau LASER de traitement modulé en au moins deux points d'impact formant un motif dans un plan de focalisation (81),
- un coupleur optique (3) entre la source LASER (1) et le système de mise en forme (2),
- un correcteur de polarisation (7) pour modifier la polarisation du faisceau LASER de traitement (11) en amont du coupleur optique (3), le correcteur de polarisation (7) étant monté entre la source LASER (1) et le système de mise en forme (2),
***caractérisé en ce que* :**
• le coupleur optique inclut une fibre à cristal photonique (31), et **en ce que**
• le correcteur de polarisation (7) est apte à modifier la polarisation du faisceau LASER de traitement (11) en amont du coupleur optique (3) de sorte que la polarisation du faisceau LASER de traitement (11) en aval du coupleur optique (3) corresponde à une polarisation de référence souhaitée, ledit correcteur de polarisation comprenant :
▪ des moyens (71) pour mesurer une variation de polarisation entre une extrémité d'entrée du coupleur optique (3) et une extrémité de sortie du coupleur optique (3), et
▪ des moyens (72) pour modifier la polarisation du faisceau LASER de traitement (11) en amont du coupleur optique (3) de sorte à compenser la variation de la polarisation mesurée.

2. Appareil de découpe selon la revendication 1, ***dans lequel*** les moyens (71) pour mesurer sont optiquement connectés à l'extrémité de sortie du coupleur optique (3), lesdits moyens (71) pour mesurer étant aptes à mesurer une variation de polarisation du faisceau LASER de traitement (11) à partir d'un faisceau LASER de mesure (12) généré par la source LASER (1), l'intensité du faisceau LASER de mesure (12) étant inférieure à l'intensité du faisceau LASER de traitement (11).

3. Appareil de découpe selon la revendication 2, ***dans lequel*** les moyens (71) pour mesurer comprennent :
- un polariseur (711) pour filtrer sélectivement un plan de polarisation du faisceau LASER de mesure (12), et
- un analyseur de polarisation (712) monté en aval du polariseur (711) pour mesurer une information représentative de la polarisation du faisceau LASER de mesure (12) en sortie du coupleur optique (3).

4. Appareil de découpe selon l'une quelconque des revendications 1 à 3, ***dans lequel*** les moyens (71) pour mesurer comprennent en outre un calculateur pour calculer, à partir de l'information mesurée par l'analyseur de polarisation (712), une variation de polarisation Δₚₒₗₐᵣᵢₛₐₜᵢₒₙ entre :
• la polarisation du faisceau LASER de mesure (12) émis par la source LASER (1), et
• la polarisation du faisceau LASER de mesure (12) reçu par l'analyseur de polarisation (712).

5. Appareil de découpe selon l'une quelconque des revendications 1 à 4, ***dans lequel*** les moyens (72) pour modifier la polarisation du faisceau LASER de traitement (11) sont disposés entre la source LASER (1) et le coupleur optique (3), lesdits moyens (72) pour modifier la polarisation étant aptes à pivoter le plan de polarisation du faisceau LASER de traitement (11) en amont du coupleur optique (3) d'un angle opposé à la variation de polarisation mesurée.

6. Appareil de découpe selon l'une des revendications 1 à 5, *dans lequel* la polarisation de référence souhaitée est égale à la polarisation du faisceau LASER de traitement (11) en sortie de la source LASER (1) (i.e. préalablement à la traversée du correcteur de polarisation (7) et du coupleur optique (3)).

7. Appareil de découpe selon l'une quelconque des revendications 1 à 6, *lequel* comprend en outre :
- un scanner optique de balayage (4), positionné en aval du système de mise en forme (2), pour déplacer le motif le long d'un chemin de déplacement prédéfini dans le plan de focalisation (81),
- un système optique de focalisation (5), positionné en aval du scanner optique de balayage (4), pour déplacer le plan de focalisation (81) du faisceau LASER modulé dans un plan de découpe du tissu (7) désiré,
- une unité de commande (6) pour piloter la source LASER (1), le système de mise en forme (2), le coupleur optique (3), le scanner optique de balayage (4) et le système optique de focalisation (5).

8. Dispositif de thérapie comportant un caisson (210) et un bras articulé (200) monté sur le caisson (210), le bras (200) incluant plusieurs segments de bras (201-204) reliés par des articulations (205-207), ***caractérisé en ce que*** le dispositif de thérapie comprend en outre un appareil de découpe selon l'une quelconque des revendications 1 à 7, le système de mise en forme (2), le scanner optique de balayage (4) et le système optique de focalisation (5) étant montés dans un segment d'extrémité (204) du bras articulé (200), la source LASER (1) et l'unité de commande (6) étant intégrées au caisson (210).

9. Dispositif de thérapie selon la revendication 8, ***dans lequel*** les moyens (72) pour modifier la polarisation du faisceau LASER de traitement (11) sont intégrés au caisson (210).

## Patentansprüche

1. Vorrichtung zum Schneiden von menschlichem oder tierischem Gewebe wie eine Hornhaut oder eine Linse, wobei die Vorrichtung enthält:
- eine LASER-Quelle (1) zum Senden eines LASER-Behandlungsstrahls (11) in Form von Impulsen,
- ein Formgebungssystem (2) - wie etwa einen räumlichen Lichtmodulator (SLM) -, das nach der LASER-Quelle (1) positioniert ist, um den LASER-Behandlungsstrahl (11) in einen einzigen phasenmodulierten LASER-Behandlungsstrahl umzuwandeln, wobei das Formgebungssystem (2) imstande ist, die Phase der Wellenfront des LASER-Behandlungsstrahls (11) gemäß einem Modulationssollwert zu modulieren, die berechnet ist, um die Energie des einzigen modulierten LASER-Behandlungsstrahls auf mindestens zwei Auftreffpunkte zu verteilen, die in einer Fokussierebene (81) ein Muster bilden,
- einen optischen Koppler (3) zwischen der LASER-Quelle (1) und dem Formgebungssystem (2),
- einen Polarisationskorrektor (7) zum Ändern der Polarisation des LASER-Behandlungsstrahls (11) vor dem optischen Koppler (3), wobei der Polarisationskorrektor (7) zwischen der LASER-Quelle (1) und dem Formgebungssystem (2) angebracht ist,
**dadurch gekennzeichnet, dass**:
• der optische Koppler eine photonische Kristallfaser (31) einschließt, und dadurch, dass
• der Polarisationskorrektor (7) imstande ist, die Polarisation des LASER-Behandlungsstrahls (11) vor dem optischen Koppler (3) zu ändern, so dass die Polarisation des LASER-Behandlungsstrahls (11) nach dem optischen Koppler (3) einer gewünschten Referenzpolarisation entspricht, wobei der Polarisationskorrektor umfasst:
▪ Mittel (71) zum Messen einer Änderung der Polarisation zwischen einem Eingangsende des optischen Kopplers (3) und einem Ausgangsende des optischen Kopplers (3), und
▪ Mittel (72) zum Ändern der Polarisation des LASER-Behandlungsstrahls (11) vor dem optischen Koppler (3), um die gemessene Änderung der Polarisation zu kompensieren.

2. Schneidvorrichtung nach Anspruch 1, wobei die Messmittel (71) optisch mit dem Ausgangsende des optischen Kopplers (3) verbunden sind, wobei die Messmittel (71) imstande sind, eine Änderung der Polarisation des LASER-Behandlungsstrahls (11) ausgehend von einem von der LASER-Quelle (1) erzeugten LASER-Messstrahl (12) zu messen, wobei die Stärke des LASER-Messstrahls (12) kleiner ist als die Stärke des LASER-Behandlungsstrahls (11).

3. Schneidvorrichtung nach Anspruch 2, wobei die Messmittel (71) umfassen:
- einen Polarisator (711) zum selektiven Filtern einer Polarisationsebene des LASER-Messstrahls (12), und
- einen Polarisationsanalysator (712), der nach dem Polarisator (711) angebracht ist, um eine Information zu messen, die für die Polarisation des LASER-Messstrahls (12) am Ausgang des optischen Kopplers (3) repräsentativ ist.

4. Schneidvorrichtung nach einem der Ansprüche 1 bis 3, wobei die Messmittel (71) ferner einen Rechner umfassen, um ausgehend von der vom Polarisationsanalysator (712) gemessenen Information eine Polarisationsänderung Δ_{Polarisation} zu berechnen zwischen:
• der Polarisation des von der LASER-Quelle (1) gesendeten LASER-Messstrahls (12), und
• der Polarisation des vom Polarisationsanalysator (712) empfangenen LASER-Messstrahls (12).

5. Schneidvorrichtung nach einem der Ansprüche 1 bis 4, wobei die Mittel (72) zum Ändern der Polarisation des LASER-Behandlungsstrahls (11) zwischen der LASER-Quelle (1) und dem optischen Koppler (3) angeordnet sind, wobei die Mittel (72) zum Ändern der Polarisation imstande sind, die Polarisationsebene des LASER-Behandlungsstrahls (11) vor dem optischen Koppler (3) um einen Winkel zu schwenken, der zur gemessenen Polarisationsänderung entgegengesetzt ist.

6. Schneidvorrichtung nach einem der Ansprüche 1 bis 5, wobei die gewünschte Referenzpolarisation gleich der Polarisation des LASER-Behandlungsstrahls (11) am Ausgang der LASER-Quelle (1) ist (das heißt vor dem Durchgang durch den Polarisationskorrektor (7) und den optischen Koppler (3)).

7. Schneidvorrichtung nach einem der Ansprüche 1 bis 6, die ferner umfasst:
- einen optischen Abtastscanner (4), der nach dem Formgebungssystem (2) positioniert ist, um das Muster entlang eines vordefinierten Bewegungspfads in der Fokussierebene (81) zu verlagern,
- ein optisches Fokussiersystem (5), das nach dem optischen Abtastscanner (4) positioniert ist, um die Fokussierebene (81) des modulierten LASER-Strahls in einer gewünschten Schnittebene des Gewebes (7) zu verlagern,
- eine Steuereinheit (6) zum Steuern der LASER-Quelle (1), des Formgebungssystems (2), des optischen Kopplers (3), des optischen Abtastscanners (4) und des optischen Fokussiersystems (5) .

8. Therapievorrichtung mit einem Kasten (210) und einem an dem Kasten (210) angebrachten Gelenkarm (200), wobei der Arm (200) mehrere Armsegmente (201-204) enthält, die durch Gelenke (205-207) verbunden sind, **dadurch gekennzeichnet, dass** die Therapievorrichtung ferner eine Schneidvorrichtung nach einem der Ansprüche 1 bis 7 umfasst, wobei das Formgebungssystem (2), der optische Abtastscanner (4) und das optische Fokussiersystem (5) in einem Endsegment (204) des Gelenkarms (200) angebracht sind, wobei die LASER-Quelle (1) und die Steuereinheit (6) in dem Gehäuse (210) integriert sind.

9. Therapievorrichtung nach Anspruch 8, wobei die Mittel (72) zum Ändern der Polarisation des LASER-Behandlungsstrahls (11) in dem Gehäuse (210) integriert sind.

## Claims

1. An apparatus for cutting human or animal tissue, such as a cornea, or a lens, said apparatus including:
- a laser source (1) for emitting a treatment laser beam (11) in the form of pulses,
- a shaping system (2) - such as a spatial light modulator (SLM) - positioned downstream of the laser source (1), for transforming the treatment laser beam (11) into a single phase-modulated treatment laser beam, the shaping system (2) being able to modulate the phase of the wavefront of the treatment laser beam (11) according to a modulation set value calculated to distribute the energy of the single modulated treatment laser beam into at least two impact points forming a pattern in a focusing plane (81),
- an optical coupler (3) between the laser source (1) and the shaping system (2),
- a polarization corrector (7) for modifying the polarization of the treatment laser beam (11) upstream of the optical coupler (3), the polarization corrector (7) being mounted between the laser source (1) and the shaping system (2),
***characterized in that*:**
• the optical coupler includes a photonic crystal fiber (31), and **in that**
• the polarization corrector (7) is configured to modify the polarization of the treatment laser beam (11) upstream of the optical coupler (3) so that the polarization of the treatment laser beam (11) downstream of the optical coupler (3) corresponds to a desired reference polarization, said polarization corrector comprising:
▪ means (71) for measuring a polarization variation between an input end of the optical coupler (3) and an output end of the optical coupler (3), and
▪ means (72) for modifying the polarization of the treatment laser beam (11) upstream of the optical coupler (3) so as to compensate for the measured polarization variation.

2. The cutting apparatus according to claim 1, ***wherein*** the measuring means (71) are optically connected to the output end of the optical coupler (3), said measuring means (71) being able to measure a polarization variation of the treatment laser beam (11) from a measurement laser beam (12) generated by the laser source (1), the intensity of the measurement laser beam (12) being lower than the intensity of the treatment laser beam (11).

3. The cutting apparatus according to claim 2, ***wherein*** the measuring means (71) comprise:
- a polarizer (711) for selectively filtering a polarization plane of the measurement laser beam (12), and
- a polarization analyzer (712) mounted downstream of the polarizer (711) for measuring information representative of the polarization of the measurement laser beam (12) at the output of the optical coupler (3).

4. The cutting apparatus according to any one of claims 1 to 3, ***wherein*** the measuring means (71) further comprise a computer for calculating, from the information measured by the polarization analyzer (712), a polarization variation Δ_{polarization} between:
• the polarization of the measurement laser beam (12) emitted by the laser source (1), and
• the polarization of the measurement laser beam (12) received by the polarization analyzer (712).

5. The cutting apparatus according to any one of claims 1 to 4, ***wherein*** the means (72) for modifying the polarization of the treatment laser beam (11) are disposed between the laser source (1) and the optical coupler (3), said means (72) for modifying the polarization being able to pivot the polarization plane of the treatment laser beam (11) upstream of the optical coupler (3) by an angle opposite to the measured polarization variation.

6. The cutting apparatus according to any of claims 1 to 5, ***wherein*** the desired reference polarization is equal to the polarization of the treatment laser beam (11) at the output of the laser source (1) (i.e. before traveling through the polarization corrector (7) and the optical coupler (3)).

7. The cutting apparatus according to any one of claims 1 to 6, ***which*** further comprises:
- an sweeping optical scanner (4), positioned downstream of the shaping system (2), for moving the pattern along a predefined movement path in the focusing plane (81),
- an optical focusing system (5), positioned downstream of the sweeping optical scanner (4), for moving the focusing plane (81) of the modulated laser beam in a cutting plane for the desired tissue (7),
- a control unit (6) for driving the laser source (1), the shaping system (2), the optical coupler (3), the sweeping optical scanner (4) and the optical focusing system (5).

8. A therapy device including a casing (210) and a hinged arm (200) mounted on the casing (210), the arm (200) including a plurality of arm segments (201-204) connected by hinges (205-207), ***characterized in that*** the therapy device further comprises a cutting apparatus according to any one of claims 1 to 7, the shaping system (2), the sweeping optical scanner (4) and the optical focusing system (5) being mounted in an end segment (204) of the hinged arm (200), the laser source (1) and the control unit (6) being integrated into the casing (210).

9. The therapy device according to claim 8, ***wherein*** the means (72) for modifying the polarization of the treatment laser beam (11) are integrated into the casing (210).
